# EUROPEAN PATENT SPECIFICATION

(11) **EP 2 806 801 B1**
(45) Date of publication and mention of the grant of the patent: **29.11.2017**
(21) Application number: 13713965.5
(22) Date of filing: 22.01.2013
(51) Int. Cl.: A61B 6/14, A61B 6/00, A61B 6/03, G06T 11/00, G06T 5/00

(54) **METAL ARTIFACT REDUCTION FROM METALLIC DENTAL FILLINGS AND/OR CROWNS IN COMPUTED TOMOGRAPHY (CT) AND/OR X-RAY**
VERRINGERUNG VON METALLARTEFAKTEN AUS METALLISCHEN ZAHNFÜLLUNGEN UND/ODER KRONEN IN DER COMPUTERTOMOGRAFIE (CT) UND/ODER BEI RÖNTGENSTRAHLEN
RÉDUCTION D'ARTEFACTS MÉTALLIQUES PROVENANT DE COURONNES ET/OU DE PRODUITS D'OBTURATION DENTAIRE EN MÉTAL EN TOMOGRAPHIE PAR ORDINATEUR (CT) ET/OU RADIOGRAPHIE

(30) Priority: 25.01.2012 US 201261590354 P
(43) Date of publication of application: 03.12.2014
(73) Proprietor: Koninklijke Philips N.V., 5656 AE Eindhoven (NL); Philips Deutschland GmbH, 20099 Hamburg (DE)
(72) Inventor: KOEHLER, Thomas, NL-5656 AE Eindhoven (NL)
(74) Representative: Steffen, Thomas
(86) International application number: PCT/IB2013/050544
(87) International publication number: WO 2013/111052

(56) References cited:
- US-A- 3 722 101
- US-A- 4 945 553
- US-A- 5 009 595
- US-A- 5 735 691
- P. HENROT ET AL: "Dynamic Maneuvers in Local Staging of Head and Neck Malignancies with Current Imaging Techniques: Principles and Clinical Applications", RADIOGRAPHICS, vol. 23, no. 5, 1 September 2003 (2003-09-01), pages 1201-1213, XP055062592, ISSN: 0271-5333, DOI: 10.1148/rg.235025045
- A. Cistaro ET AL: "Assessment of a New 18F-FDG PET/CT Protocol in the Staging of Oral Cavity Carcinomas", JOURNAL OF NUCLEAR MEDICINE TECHNOLOGY., vol. 39, no. 1, 14 February 2011 (2011-02-14), pages 7-13, XP055240201, US ISSN: 0091-4916, DOI: 10.2967/jnmt.110.074906

## Description

The following generally relates to imaging, more particularly to reducing metal artifact in images from metallic dental fillings and/or crowns, finds particular application to computed tomography (CT), and is also amenable to other imaging modalities such as x-ray.

A CT scanner includes an x-ray tube mounted on a rotatable gantry that rotates around an examination region about a z-axis. A detector array subtends an angular arc opposite the examination region across from the x-ray tube. For a scan, the x-ray tube emits radiation that traverses the examination region and a portion of an object or subject therein. The detector array detects radiation that traverses the examination region and the portion of an object or subject, and generates projection data indicative thereof. A reconstructor reconstructs the projection data, generating volumetric image data indicative thereof. The volumetric image data can be processed to generate one or more images indicative of the scanned portion of the scanned object or subject.

With CT, as well as other imaging modalities, various artifacts can degrade the visual and diagnostic quality of the generated images. One source of such artifact is metallic material in the scanned portion of the scanned object or subject. Other sources of artifact include the imaging system, the image processing system, and/or other sources. Generally, metallic materials result in "streak" artifact, which occurs because filtered backprojection (FBP) algorithms assume that each detector measurement is equally accurate, when, in practice, x-ray beams that pass through or near metallic materials are highly attenuated relative to x-ray beams that do not pass through or near metallic materials.

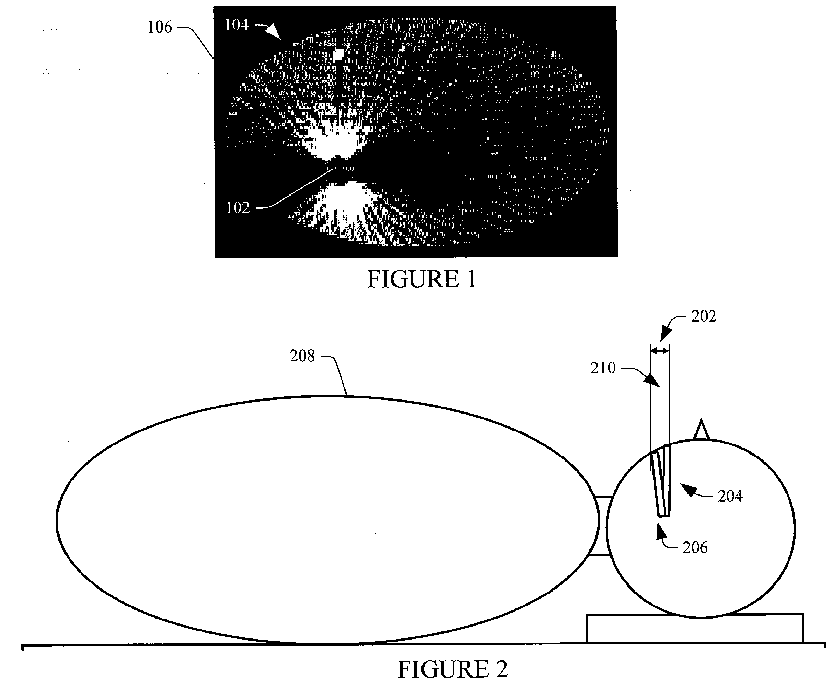

An example of such artifact caused by a metallic material is shown in FIGURE 1. In FIGURE 1, a metallic object 102 causes streaks 104 in a backprojected image 106, as well as dark or bright shading artifacts. Metal artifact reduction techniques exist. However, the results are often not suitable, in particular, if there are a lot of metallic objects within the field of view because large parts of the sinogram are affected. This situation is often present when the jaw with dental fillings is in the field of view. Typically, all dental fillings present in the upper or lower jaw are visible in the same slice, and if there are fillings or crowns on the incisors, the metal from both jaws may even be present in the same axial slices.

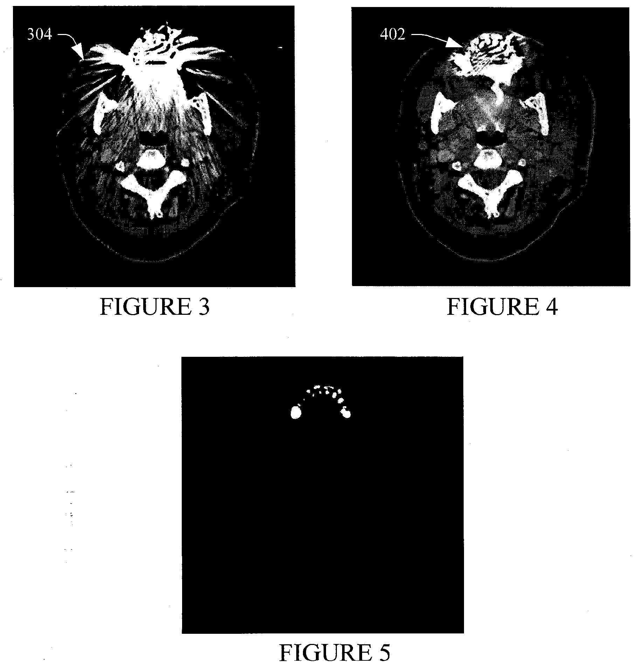

By way of example, FIGURE 2 shows a z-extent 202 for an example head scan which covers both jaws 204 and 206 of a subject 208 where dental fillings of the subject 208 are scanned in the same acquisition interval 210, for purposes of explanation. FIGURE 3 shows a reconstructed image with streak artifact (e.g. a streak 304 of a plurality of streaks) caused by the metallic fillings in teeth of the jaws 204 and 206. FIGURE 4 shows the same image after applying a metal artifact reduction algorithm. Note that resulting image still includes artifact 402 and may not be suitable for diagnostic purposes. For reference, FIGURE 5 shows an image created by segmenting the metallic fillings from the image of FIGURE 3. As can be seen, in this example, multiple metallic fillings are in the same image.

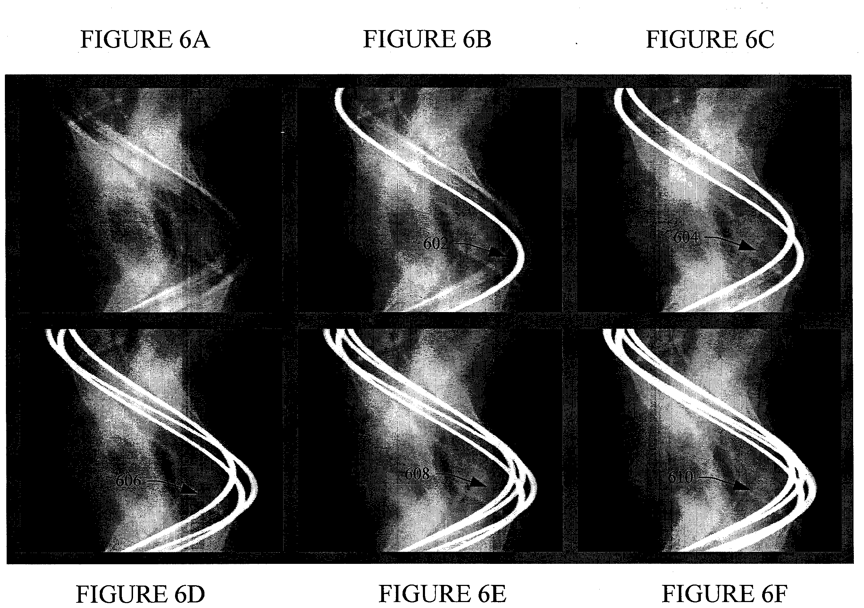

FIGURES 6A-6F respectfully show sinograms with increasing amount of metal shadow (e.g., 602, 604, 606, 608 and 610) caused by an increasing number of metallic filings in the acquired data. In this example, the number of metallic fillings increases from zero (0) to six (6) metallic fillings going from FIGURE 6A to FIGURE 6F. Unfortunately, the interpolation required across the shadow becomes increasingly more demanding with the increasing number of metallic fillings, and, as shown in FIGURE 4, may not be able to be suitably removed from the resulting images. The study "Assessment of a new F-FDG PET/CT Protocol in the Staging of Oral Cavity Carcinomas" studies the evaluation of the extent of the lesion in oral carcinoma and among other parameters also studies the effect of scanning with open-mouth. The study "Dynamic Maneuvers in Local Staging of Head and Neck Malignancies With Current Imaging Techniques: Principles and Clinical Applications" studies also the open-mouth technique.

Aspects described herein address the above-referenced problems and others.

The present invention is defined in the appended independent claims 1 and 8.

In one aspect of the present disclosure, a method includes planning a head scan of a subject via a console of an imaging system. The head scan scans both jaws of the subject, including metallic dental fillings of teeth of the subject. The method further includes scanning the subject, including the metallic dental fillings of the teeth of the subject, via the imaging system. During the scan, the subject has a dental metal artifact reduction device in their mouth. The method further includes generating projection data indicative of the scanned region of the head of the subject, including the metallic dental fillings of the teeth of the subject.

In another aspect of the present disclosure, a dental metal artifact reduction device includes sides and a region therebetween. The sides position the jaws of a subject for a scan such that metallic dental fillings of the teeth of the subject are distributed along a z-extent in which at least some of the metallic dental fillings are imaged during different data acquisition intervals of the scan.

In another aspect of the present disclosure, provided for explanation only, a method includes installing a dental metal artifact reduction device in the mouth of a subject to be scanned, wherein the installed dental metal artifact reduction device separates the jaws of the subject by a predetermined amount. The method further includes planning a head scan of the subject via a console of an imaging system, wherein the head scan scans both of the jaws of the subject, including the metallic dental fillings of teeth of the subject. The method further includes scanning the subject, including the metallic dental fillings of the teeth of the subject, via the imaging system, wherein the subject has a dental metal artifact reduction device installed in their mouth during the scan. The method further includes generating projection data indicative of the scanned region of the head of the subject, including the metallic dental fillings of the teeth of the subject.

The drawings are only for purposes of illustrating the preferred embodiments and are not to be construed as limiting the invention.
FIGURE 1 illustrates streak and shading artifacts in an image caused by a metallic structure in a scanned object.
FIGURE 2 schematically illustrates an example head scan, showing the z-extent of an acquisition.
FIGURE 3 shows a reconstructed image of a head scan over the z-extent of FIGURE 2 with streak artifact caused by metallic dental fillings.
FIGURE 4 shows the results of processing the reconstructed image of FIGURE 3 with a metal artifact reduction algorithm.
FIGURE 5 shows a segmentation of the metallic fillings of the reconstructed image of FIGURE 5.
FIGURES 6A-6F shows sinograms with shadows for acquisitions covering zero (0) to six (6) metallic fillings.
FIGURE 7 schematically illustrates an imaging system in connection with a dental metal artifact device.
FIGURE 8 schematically illustrates an example head scan, showing the z-extent of an acquisition using the dental metal artifact reduction device.
FIGURE 9 illustrates an example method using the dental metal artifact reduction device described herein.
FIGURE 10 illustrates another example method using the dental metal artifact reduction device described herein.

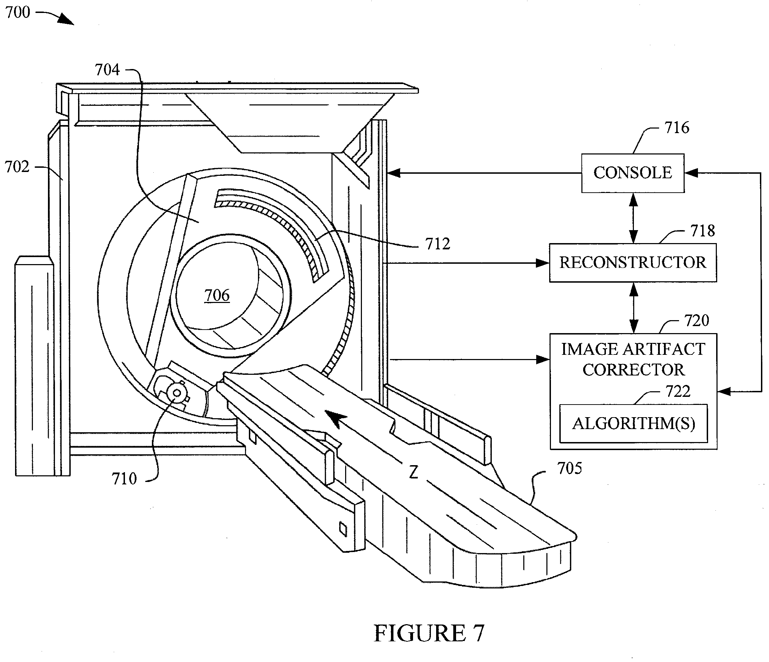

FIGURE 7 illustrates an example imaging system 700, such as a computed tomography (CT) scanner. The imaging system 700 includes a generally stationary gantry 702 and a rotating gantry 704. The rotating gantry 704 is rotatably supported by the stationary gantry 702 and rotates around an examination region 706 about a z-axis. A subject support 705, such as a couch, supports an object or subject in the examination region 706. In one non-limiting instance, the subject is scanned using a head or other imaging protocol in which metallic dental fillings of the subject are in the examination region during scanning.

A radiation source 710, such as an x-ray tube, is rotatably supported by the rotating gantry 704. The radiation source 710 rotates with the rotating gantry 704 and emits radiation that traverses the examination region 706 and a sub-portion of the object or subject therein. A one or two dimensional radiation sensitive detector array 712 subtends an angular arc opposite the radiation source 710 across the examination region 706. The detector array 712 detects radiation traversing the examination region 706 and the sub-portion of the object or subject, and generates projection data indicative thereof.

A general-purpose computing system or computer serves as an operator console 716. The console 716 includes a human readable output device such as a monitor and an input device such as a keyboard, mouse, etc. Software resident on the console 716 allows the operator to interact with and/or operate the scanner 700 via a graphical user interface (GUI) or otherwise. Such interaction may include selecting an imaging protocol such as a head protocol, selecting an image artifact removal algorithm, etc. A reconstructor 718 reconstructs the projection data and generates volumetric image data indicative thereof.

An artifact corrector 720 employs at least one metal artifact correction algorithm 722 to correct for streak artifact caused by a metallic object (e.g., a metallic dental filling and/or crown) in the examination region 706 during scanning. In one instance, the artifact corrector 720 employs an image domain algorithm which corrects for the streak artifact in the image domain. In another instance, the artifact corrector 720 employs a projection domain algorithm which corrects for the streak artifact in the projection domain. In yet another instance, the artifact corrector 720 employs an algorithm which goes back and forth between the projection and image domains to correct for the streak artifact.

An example of a suitable metal artifact reduction approach is described in US patent 7,340,027 B2, filed June 16, 2004, and entitled "Metal Artifact Correction in Computed Tomography". Another example of a suitable metal artifact reduction approach is described in PCT publication WO 2008/084352 A1, filed December 19, 2007, and entitled "Apparatus, Method and Computer Program for Producing a Corrected Image of a Region of Interest from Acquired Projection Data". Other approaches are also contemplated herein.

As briefly discussed above, in one non-limiting instance, the subject is scanned using a head or other imaging protocol in which metallic dental fillings (and/or crowns) of the subject are in the examination region during scanning. Also discussed above, metallic dental fillings (and/or crowns), when located in the examination region 706 during a scan, may cause streak artifact (e.g., as shown in FIGURE 3) that cannot be corrected very well by known metal artifact reduction techniques (e.g., as demonstrated in FIGURE 4), especially where multiple filling (and/or crowns) are concurrently present in the examination region 706 such that they are present in the same sinogram and image of the reconstructed image data.

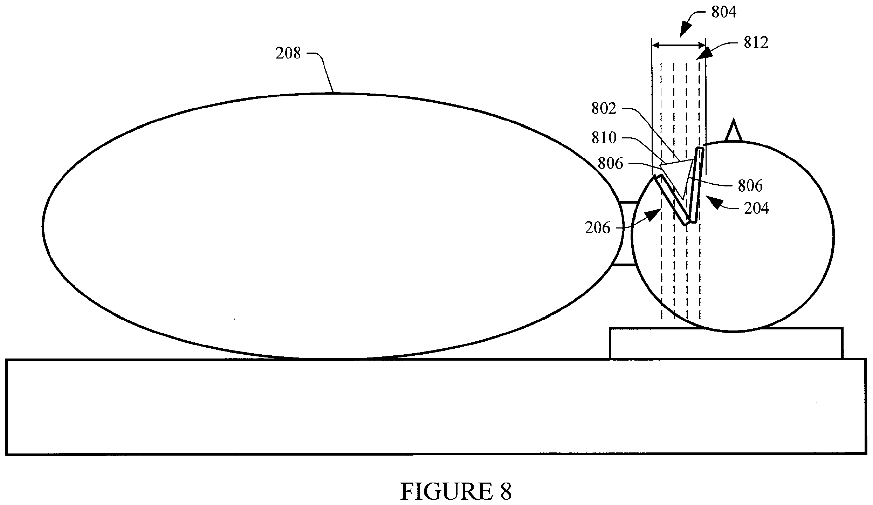

FIGURE 8 shows an embodiment in which a dental metal artifact reduction device 802 is utilized in connection with the subject 208 to position the jaws 204 and 206 of the subject with respect to each other so that metallic dental fillings (and/or crowns) of the subject 208, for a z-extent 804, are distributed over multiple data acquisition intervals 812 (and hence images) relative to FIGURE 2, where the same data acquisition interval 210 images all of the fillings (and/or crowns). In this manner, some of the metallic dental fillings (and/or crowns) are imaged during different data acquisition intervals, and the sinogram corresponding to any of the multiple data acquisition intervals will have less shadowing (e.g., compare FIGURE 6A and FIGURE 6F), such that the interpolation required across the shadow is not too demanding for conventional metal artifact correction algorithms to suitable remove metal artifacts.

In the example, the dental metal artifact reduction device 802 is wedge-shaped (or shaped to easily and comfortably slide into an open mouth of the subject 108). The device 802 includes a first side 806 and a second side 808 separated by a region 810 which generally increases in width, from a point at which the two sides converge, along a transverse direction of the device 802, which is perpendicular to the z-direction of the device 802, forming the wedge. In the illustrated embodiment, the wedge has linear sides which are equal in length. However, in other embodiment, at least one of the sides can be curved or otherwise shaped, and the sides do not have to be increasingly separated or have the same length. Where the device 802 is specific to the subject, one or both of the sides may include recesses corresponding to the teeth. This may facilitate placement and/or maintaining placement in the mouth.

The geometry of the illustrated wedge is such that an installed dental metal artifact reduction device 802 fills the void or space between the palate and the tongue, which mitigates tongue motion and thus motion artifact caused by a moving tongue during the scan. That is, the dental artifact reduction device has a geometry that inhibits movement of the tongue of the subject when in the mouth of the subject during the scan. However, it is to be understood that the illustrated geometry (i.e., shape and size) of the dental metal artifact reduction device 802 is provided for explanatory purposes and is not limiting. As such, in other embodiments, the dental metal artifact reduction device 802 may have other geometries. Although the dental metal artifact reduction device 802 is shown as a single element, it is to be appreciated that is may include multiple elements, which are assembled to form the device 802, which are individually placed in the mouth of the subject 208, and/or are otherwise employed in connection with each other.

Moreover, the dental metal artifact reduction device 802 includes a material that is generally radiolucent, e.g. made of Rohacell, such that x-rays traversing the dental metal artifact reduction device 802 are substantially unattenuated. In one instance, this results in images that, visually, with respect to intensity, are similar to images acquired without the dental metal artifact reduction device 802. Less radiolucent material can alternatively be used. It is to be appreciated that a single generic dental metal artifact reduction device 802 can be used with all subjects, multiple different generic dental metal artifact reduction devices 802 can correspond to different types or categories of subject (e.g., adult, child, and infant), a single dental metal artifact reduction device 802 can be specific to a particular subject, etc. In one instance, the device 802 includes multiple members which are removably affixed to each other, and one or more of the members may be removed or added to the device 802 to change the geometry of the device 802.

In one instance, the operator of the imaging system 700, when planning a head scan for the subject 208, enters, via the console 716, information about the dental metal artifact reduction device 802. Such information can be used to automatically populate and/or recommend one or more parameters of the plan. For instance, the information my include a serial number or unique identifier which is used to obtain a length of the dental metal artifact reduction device 802 in the z-direction, and this information can be used to set the z-extent, a number of slices to reconstruct, a slice width, a metal artifact reduction algorithm 722, etc.

In another instance, where the dental metal artifact reduction device 802 includes a wireless transmitter (e.g., an RFID tag) and the console 716 includes a corresponding wireless receiver (e.g., an RFID receiver), the information about the dental metal artifact reduction device 802 and/or the corresponding subject can be transmitted from the dental metal artifact reduction device 802 to the console 716. Again, this information can be used to automatically populate and/or recommend one or more parameters of the plan.

In another instance, after planning the scan, the system 700 visually displays a serial number, name and/or unique identifier of a dental metal artifact reduction device 802 which the system 700 recommends to use for the scan. For example, the system 700 may recommend a particular dental metal artifact reduction device 802 based on the length of the dental metal artifact reduction device 802 in the z-direction in the plan.

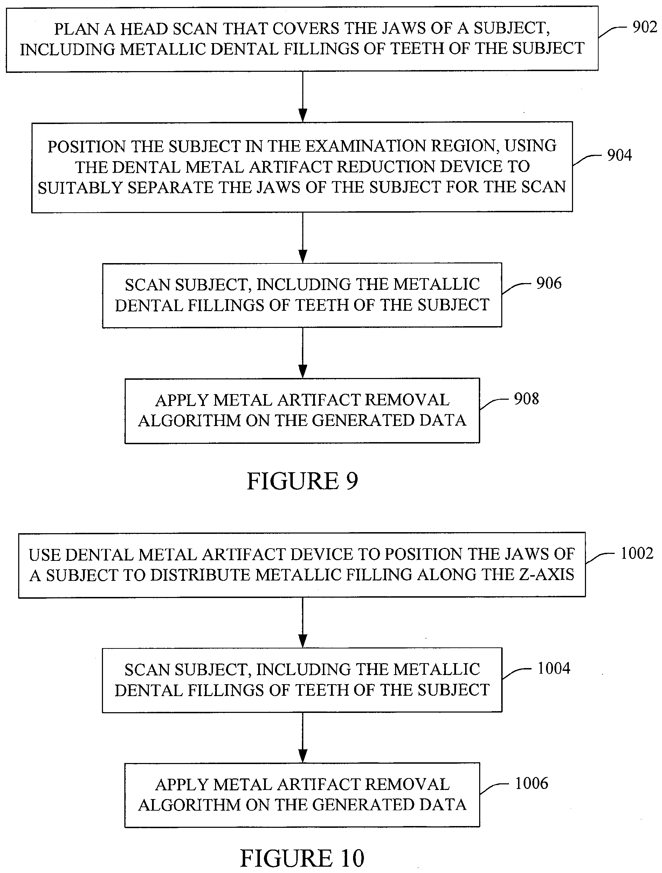

FIGURE 9 illustrates an example method.

It is to be appreciated that the ordering of the acts in the methods described herein is not limiting. As such, other orderings are contemplated herein. In addition, one or more acts may be omitted and/or one or more additional acts may be included.

At 902, a head scan, which covers the jaws 204 and 206 of the subject 208, including the metallic dental fillings of the subject 108, is planned for the subject 208 via the console 716.

At 904, the subject 208 is positioned in the examination region 706 via the subject support 705 based on planned scan start and end points.

In this example, the dental metal artifact reduction device 802 is in the mouth of the subject 208, positioning the jaws 204 and 206 such that the metallic dental fillings of the subject 208 are distributed along a z-extent that is longer than a z-extent if the jaws 204 and 206 were not separated as such, distributing the metallic dental fillings over more data acquisition internals and hence reconstructed images.

At 906, the subject 108 is scanned via the imaging system 700 based on the planned head scan, including scanning the jaws 204 and 206 and the metallic dental fillings of the teeth therein.

At 908, the projection and/or image data from the scan is processed via the image artifact corrector 720 to remove metal artifact using one of the metal reduction artifact algorithms 722 as discussed herein.

FIGURE 10 illustrates another example method.

It is to be appreciated that the ordering of the acts in the methods described herein is not limiting. As such, other orderings are contemplated herein. In addition, one or more acts may be omitted and/or one or more additional acts may be included.

At 1002, the dental metal artifact reduction device 802 is placed in the mouth of the subject 108, positioning the jaws 204 and 206 such that the metallic dental fillings of the subject 108 are distributed along a predetermined z-extent that is longer than a z-extent if the jaws 204 and 206 were not separated, distributing the metallic dental fillings over more data acquisition internals and hence reconstructed images.

At 1004, the subject 108 is scanned via the imaging system 700, including the jaws 204 and 206 and the metallic dental fillings of the teeth therein.

At 1006, the projection and/or image data from the scan is processed via the image artifact corrector 720 to remove metal artifact using one of the metal reduction artifact algorithms 722 as discussed herein.

The invention has been described with reference to the preferred embodiments. Modifications and alterations may occur to others upon reading and understanding the preceding detailed description. It is intended that the invention be constructed as including all such modifications and alterations insofar as they come within the scope of the appended claims.

## Claims

1. A method, comprising:
a) planning a head scan of a subject via a console (716) of an imaging system (700), wherein the head scan scans both jaws of the subject, including metallic dental fillings of teeth of the subject;
b) scanning the subject, including the metallic dental fillings of the teeth of the subject, via the imaging system, wherein the subject has a dental metal artifact reduction device (802) installed in the mouth during the scan for separating the jaws of the subject; and
c) generating projection data indicative of the scanned region of the head of the subject, including the metallic dental fillings of the teeth of the subject;
**characterized in that** the dental metal artifact reduction device is wedge shaped and has a geometry that inhibits movement of the tongue of the subject when the dental metal artifact reduction device is installed in the mouth of the subject; and and **in that**:
i) the dental metal artifact reduction device (802) includes a wireless transmitter and the console (716) includes a corresponding wireless receiver; and wherein step a) further comprises automatically populating and/or recommending one or more parameters of the head scan plan based on information about the dental metal artifact reduction device (802) transmitted by the dental metal artifact reduction device to the console (716);
or
ii) an operator of the imaging system (700), when planning the head scan for the subject (208), enters, via the console (716), information about the dental metal artifact reduction device (802); and wherein step a) further comprises automatically populating and/or recommending one or more parameters of the head scan plan based on the information about the dental metal artifact reduction device (802).

2. The method of claim 1, further comprising:
applying a projection domain metal artifact reduction algorithm to the projection data, thereby reducing metal artifact caused by the metallic dental fillings.

3. The method of claim 1 or 2, further comprising:
reconstructing the projection data, producing image data; and
applying an image domain metal artifact reduction algorithm to the image data, thereby reducing metal artifact caused by the metallic dental fillings.

4. The method of any of claims 1 to 3, wherein the dental metal artifact reduction device includes a material which is substantially radiolucent.

5. The method of any of claims 1 to 4, wherein a single generic dental metal artifact reduction device is used for all subjects.

6. The method of any of claims 1 to 4, wherein the dental metal artifact reduction device is specific to an individual subject.

7. The method of any of claims 1 to 4, wherein the dental metal artifact reduction device is one of a plurality of metal artifact reduction devices, each corresponding to a different class of subjects.

8. An imaging system (700) comprising:
- a generally stationary gantry (702) and a rotating gantry (704) rotatably supported by the stationary gantry (702), wherein an X-ray radiation source (710) and a radiation sensitive detector array (712) are rotatably supported by the rotating gantry;
- an operator console (716) which includes a human readable output device and an input device; and
- a dental metal artifact reduction device for separating the jaws of a subject when installed in the mouth of the subject;
wherein a head scan can be planned by an operator of the imaging sysetm (700) via the console (716), wherein the head scan covers both jaws of the subject, including metallic dental fillings of teeth of the subject;
wherein the imaging system is configured to scan the subject, including the metallic dental fillings of the teeth of the subject, wherein the subject has the dental metal artifact reduction device installed in the mouth during the scan for separating the jaws of the subject;
wherein the radiation sensitive detector array (712) is configured to generate projection data indicative of the scanned region of the head of the subject including the metallic dental fillings of the teeth of the subject;
**characterized in that**
the dental metal artifact reduction device (802) is wedge shaped and has a geometry that inhibits movement of the tongue of the subject when the dental metal artifact reduction device (802) is installed in the mouth of the subject;
and **in that**:
i) the console is configured receive information about the dental metal artifact reduction device (802) entered by an operator of the system (700); or
ii) the dental metal artifact reduction device (802) comprises a wireless transmitter and the console (716) comprises a corresponding wireless receiver, wherein the dental metal artifact reduction device (802) and the console (716) are configured so that information about the dental metal artifact reduction device (802) is transmitted from the dental metal artifact reduction device (802) to the console (716);
and **in that** the imaging system (700) is further configured to automatically populate and/or recommend one or more parameters of the head scan plan based on the information about the dental metal artifact reduction device.

9. The system of claim 8, wherein the dental metal artifact reduction device includes a material which is substantially radiolucent.

## Patentansprüche

1. Verfahren, umfassend:
a) Planen eines Kopf-Scans eines Patienten über eine Konsole (716) eines Bildgebungssystems (700), wobei bei dem Kopf-Scan beide Kiefer des Patienten einschließlich metallischer Zahnfüllungen der Zähne des Patienten gescannt werden;
b) Scannen des Patienten, einschließlich der metallischen Zahnfüllungen der Zähne des Patienten, über das Bildgebungssystem, wobei dem Patienten während des Scans eine dentale Metallartefaktreduzierungsvorrichtung (802) zum Trennen der Kiefer des Patienten in den Mund eingesetzt ist; und
c) Erzeugen von Projektionsdaten, die die gescante Kopfregion des Patienten, einschließlich der metallischen Zahnfüllungen des Patienten, angeben;
**dadurch gekennzeichnet, dass** die dentale Metallartefaktreduzierungsvorrichtung keilförmig ist und eine Geometrie hat, welche den Patienten am Bewegen der Zunge hindert, wenn die dentale Metallartefaktreduzierungsvorrichtung in den Mund des Patienten eingesetzt ist; und
dadurch, dass:
i) die dentale Metallartefaktreduzierungsvorrichtung (802) einen drahtlosen Sender umfasst und die Konsole (716) einen entsprechenden drahtlosen Empfänger umfasst; und wobei Schritt a) weiterhin das automatische Einpflegen und/oder Empfehlen von einem oder mehreren Parametern des Kopf-Scan-Plans basierend auf Informationen über die dentale Metallartefaktreduzierungsvorrichtung (802) umfasst, die durch die dentale Metallartefaktreduzierungsvorrichtung an die Konsole (716) gesendet werden;
oder
ii) ein Bediener des Bildgebungssystems (700) beim Planen des Kopf-Scans für den Patienten (208) über die Konsole (716) Informationen über die dentale Metallartefaktreduzierungsvorrichtung (802) eingibt; und wobei Schritt a) weiterhin das automatische Einpflegen und/oder Empfehlen von einem oder mehreren Parametern des Kopf-Scan-Plans basierend auf Informationen über die dentale Metallartefaktreduzierungsvorrichtung (802) umfasst.

2. Verfahren nach Anspruch 1, weiterhin umfassend:
Anwenden eines Projektionsbereich-Metallartefaktreduzierungsalgorithmus auf die Projektionsdaten, wodurch der durch die metallischen Zahnfüllungen verursachte Metallartefakt reduziert wird.

3. Verfahren nach Anspruch 1 oder 2, weiterhin umfassend:
Rekonstruieren der Projektionsdaten, Produzieren von Bilddaten; und
Anwenden eines Bildbereich-Metallartefaktreduzierungsalgorithmus auf die Bilddaten, wodurch der durch die metallischen Zahnfüllungen verursachte Metallartefakt reduziert wird.

4. Verfahren nach einem der Ansprüche 1 bis 3, wobei die dentale Metallartefaktreduzierungsvorrichtung ein Material umfasst, das im Wesentlichen strahlendurchlässig ist.

5. Verfahren nach einem der Ansprüche 1 bis 4, wobei eine einzige dentale Metallartefaktreduzierungsvorrichtung für alle Patienten verwendet wird.

6. Verfahren nach einem der Ansprüche 1 bis 4, wobei die dentale Metallartefaktreduzierungsvorrichtung für einen individuellen Patienten spezifisch ist.

7. Verfahren nach einem der Ansprüche 1 bis 4, wobei die dentale Metallartefaktreduzierungsvorrichtung eine von einer Vielzahl von Metallartefaktreduzierungsvorrichtungen ist, die jeweils einer anderen Patientenklasse entsprechen.

8. Bildgebungssystem (700), umfassend:
- eine im Allgemeinen stationäre Gantry (702) und eine rotierende Gantry (704), die drehbar von der stationären Gantry (702) getragen wird, wobei eine Röntgenstrahlungsquelle (710) und ein strahlungsempfindliches Detektorarray (712) drehbar von der rotierenden Gantry getragen werden;
- eine Bedienerkonsole (716), die eine durch Menschen lesbare Ausgabevorrichtung und eine Eingabevorrichtung umfasst; und
- eine dentale Metallartefaktreduzierungsvorrichtung zum Trennen der Kiefer eines Patienten, wenn sie in den Mund des Patienten eingesetzt ist;
wobei ein Kopf-Scan durch den Bediener des Bildgebungssystems (700) über eine Konsole (716) geplant werden kann, wobei der Kopf-Scan beide Kiefer des Patienten einschließlich metallischer Zahnfüllungen der Zähne des Patienten abdeckt;
wobei das Bildgebungssystem dafür konfiguriert ist, den Patienten, einschließlich der metallischen Zahnfüllungen der Zähne des Patienten, zu scannen, wobei dem Patienten während des Scans eine dentale Metallartefaktreduzierungsvorrichtung zum Trennen der Kiefer des Patienten in den Mund eingesetzt ist;
wobei das strahlungsempfindliche Detektorarray (712) dafür konfiguriert ist, Projektionsdaten zu erzeugen, die die gescante Kopfregion des Patienten, einschließlich der metallischen Zahnfüllungen des Patienten, angeben;
**dadurch gekennzeichnet, dass**
die dentale Metallartefaktreduzierungsvorrichtung (802) keilförmig ist und eine Geometrie hat, welche den Patienten am Bewegen der Zunge hindert, wenn die dentale Metallartefaktreduzierungsvorrichtung (802) in den Mund des Patienten eingesetzt ist;
und dadurch, dass:
i) die Konsole dafür konfiguriert ist, Informationen über die dentale Metallartefaktreduzierungsvorrichtung (802) zu empfangen, die von einem Bediener des Systems (700) eingegeben wurden; oder
ii) die dentale Metallartefaktreduzierungsvorrichtung (802) einen drahtlosen Sender umfasst und die Konsole (716) einen entsprechenden drahtlosen Empfänger umfasst; wobei die dentale Metallartefaktreduzierungsvorrichtung (802) und die Konsole (716) so konfiguriert sind, dass Informationen über die dentale Metallartefaktreduzierungsvorrichtung (802) von der dentalen Metallartefaktreduzierungsvorrichtung (802) an die Konsole (716) gesendet werden;
und dass das Bildgebungssystem (700) weiterhin dafür konfiguriert ist, automatisch einen oder mehrere Parameter des Kopf-Scan-Plans basierend auf Informationen über die dentale Metallartefaktreduzierungsvorrichtung einzupflegen und/oder zu empfehlen.

9. System nach Anspruch 8, wobei die dentale Metallartefaktreduzierungsvorrichtung ein Material umfasst, das im Wesentlichen strahlendurchlässig ist.

## Revendications

1. Procédé, comprenant :
a) la planification d'un balayage de tête d'un sujet par l'intermédiaire d'une console (716) d'un système d'imagerie (700), dans lequel le balayage de tête balaie les deux mâchoires du sujet, incluant les amalgames dentaires métalliques de dents du sujet ;
b) le balayage du sujet, incluant les amalgames dentaires métalliques des dents du sujet, par l'intermédiaire du système d'imagerie, dans lequel le sujet a un dispositif de réduction d'artefact métallique dentaire (802) installé dans la bouche durant le balayage pour séparer les mâchoires du sujet; et
c) la génération de données de projection indicatives de la région balayée de la tête du sujet, incluant les amalgames dentaires métalliques des dents du sujet ;
**caractérisé en ce que** le dispositif de réduction d'artefact métallique dentaire est cunéiforme et présente une géométrie qui empêche le déplacement de la langue du sujet quand le dispositif de réduction d'artefact métallique dentaire est installé dans la bouche du suj et ;
et **en ce que** :
i) le dispositif de réduction d'artefact métallique dentaire (802) inclut un émetteur sans fil et la console (716) inclut un récepteur sans fil correspondant; et dans lequel l'étape a) comprend en outre le chargement automatique et/ou la recommandation d'un ou de plusieurs paramètres du balayage de tête sur la base d'informations concernant le dispositif de réduction d'artefact métallique dentaire (802) transmises par le dispositif de réduction d'artefact métallique dentaire à la console (716) ;
ou
ii) un opérateur du système d'imagerie (700), lors de la planification du balayage de tête pour le sujet (208), entre, par l'intermédiaire de la console (716), des informations concernant le dispositif de réduction d'artefact métallique dentaire (802) ; et dans lequel l'étape a) comprend en outre le chargement automatique et/ou la recommandation d'un ou de plusieurs paramètres du balayage de tête sur la base des informations concernant le dispositif de réduction d'artefact métallique dentaire (802).

2. Procédé selon la revendication 1, comprenant en outre :
l'application d'un algorithme de réduction d'artefact métallique de domaine projection aux données de projection, réduisant ainsi l'artefact métallique causé par les amalgames dentaires métalliques.

3. Procédé selon la revendication 1 ou 2, comprenant en outre :
la reconstruction des données de projection, produisant des données d'image ; et
l'application d'un algorithme de réduction d'artefact métallique de domaine image aux données d'image, réduisant ainsi l'artefact métallique causé par les amalgames dentaires métalliques.

4. Procédé selon l'une quelconque des revendications 1 à 3, dans lequel le dispositif de réduction d'artefact métallique dentaire inclut un matériau qui est sensiblement radiotransparent.

5. Procédé selon l'une quelconque des revendications 1 à 4, dans lequel un dispositif de réduction d'artefact métallique dentaire générique unique est utilisé pour tous les sujets.

6. Procédé selon l'une quelconque des revendications 1 à 4, dans lequel le dispositif de réduction d'artefact métallique dentaire est spécifique à un sujet individuel.

7. Procédé selon l'une quelconque des revendications 1 à 4, dans lequel le dispositif de réduction d'artefact métallique dentaire est l'un d'une pluralité de dispositifs de réduction d'artefact métallique, chacun correspondant à une classe de sujets différente.

8. Système d'imagerie (700) comprenant :
- un portique généralement fixe (702) et un portique rotatif (704) supporté en rotation par le portique fixe (702), dans lequel une source de rayonnement à rayons X (710) et un réseau de détecteur sensibles au rayonnement (712) sont supportés en rotation par le portique rotatif ;
- une console opérateur (716) qui inclut un dispositif de sortie lisible par l'humain et un dispositif d'entrée ; et
- un dispositif de réduction d'artefact métallique dentaire pour séparer les mâchoires d'un sujet lorsqu'il est installé dans la bouche du sujet ;
dans lequel un balayage de tête peut être planifié par un opérateur du système d'imagerie (700) par l'intermédiaire de la console (716), dans lequel le balayage de tête couvre les deux mâchoires du sujet, incluant des amalgames dentaires métalliques de dents du sujet ;
dans lequel le système d'imagerie est configuré pour balayer le sujet, incluant les amalgames dentaires métalliques des dents du sujet, dans lequel le sujet a le dispositif de réduction d'artefact métallique dentaire installé dans la bouche durant le balayage pour séparer les mâchoires du sujet ;
dans lequel le réseau de détecteurs sensibles au rayonnement (712) est configuré pour générer des données de projection indicatives de la région balayée de la tête du sujet incluant les amalgames dentaires métalliques des dents du sujet ;
**caractérisé en ce que**
le dispositif de réduction d'artefact métallique dentaire (802) est cunéiforme et présente une géométrie qui empêche le déplacement de la langue du sujet quand le dispositif de réduction d'artefact métallique dentaire (802) est installé dans la bouche du suj et ;
et **en ce que** :
i) la console est configurée pour recevoir des informations concernant le dispositif de réduction d'artefact métallique dentaire (802) entrées par un opérateur du système (700) ; ou
ii) le dispositif de réduction d'artefact métallique dentaire (802) inclut un émetteur sans fil et la console (716) inclut un récepteur sans fil correspondant, dans lequel le dispositif de réduction d'artefact métallique dentaire (802) et la console (716) sont configurés de telle sorte que des informations concernant le dispositif de réduction d'artefact métallique dentaire (802) sont transmises depuis le dispositif de réduction d'artefact métallique dentaire (802) à la console (716) ;
et **en ce que** le système d'imagerie (700) est en outre configuré pour charger automatiquement et/ou recommander un ou plusieurs paramètres du balayage de tête sur la base des informations concernant le dispositif de réduction d'artefact métallique dentaire.

9. Système selon la revendication 8, dans lequel le dispositif de réduction d'artefact métallique dentaire inclut un matériau qui est sensiblement radiotransparent.
